# EUROPEAN PATENT APPLICATION

(11) **EP 4 697 017 A1**
(43) Date of publication of application: **18.02.2026**
(21) Application number: 25193367.7
(22) Date of filing: 01.08.2025
(51) Int. Cl.: G01N 25/56, G01N 33/00

(54) **ATMOSPHERIC MOISTURE MEASURING SYSTEM**

(30) Priority: 16.08.2024 US 202463683897 P; 02.05.2025 US 202519197751
(71) Applicant: HONEYWELL INTERNATIONAL INC., Charlotte, NC 28202 (US)
(72) Inventor: KERR, Ian, Charlotte, 28202 (US); PEARCE, Dan, Charlotte, 28202 (US); DRASAL, Zbynek, Charlotte, 28202 (US)
(74) Representative: Haseltine Lake Kempner LLP

(57) **Abstract**

An atmospheric moisture measuring system is provided that includes at least one moisture collector, at least one moisture sensor, at least one heater and at least one timer system. The at least one moisture collector is coupled to receive an airflow from a bleed system of a vehicle. Each moisture sensor is coupled to receive evaporated moisture from an associated moisture collector of the at least one moisture collector. Each heater is configured to heat an associated moisture collector to generate the evaporated moisture received by an associated moisture sensor. Each timer system is configured to selectively activate an associated heater.

## Description

### CROSS-REFERENCE TO RELATED APPLICATIONS

This Application claims priority to U.S. Provisional Application Serial No. 63/683,897, same title herewith, filed on August 16, 2024, which is incorporated in its entirety herein by reference.

### BACKGROUND

Aircraft contrails have been identified as a leading contributor to global warming in aviation. Therefore, the reduction of contrails is seen in the industry as a way to help decarbonize. Contrails are formed from an artefact of a complex reaction between a number of factors. Some of these factors are aircraft-based such as the products of combustion which in turn are the result of fuel composition and combustion conditions. Other factors relate to atmospheric conditions such as humidity (moisture), temperature and pressure in the atmosphere the aircraft is flying through. Temperature and pressure are straightforward to measure but, humidity measurements which are typically in the 5-10 ppm range, is not a straight-forward process. Inexpensive sensor technology, such as a capacitive type microelectromechanical system (MEMS) sensors can only measure moisture levels of around 500ppm to 1000ppm which is not sensitive enough by a factor of around 100X. Currently, there are several candidate humidity sensing technologies being developed to reach a precision in the 5-10 ppm range (e.g. optical based sensors), however, these candidates are typically complex and highly expensive for airborne applications.

For the reasons stated above and for other reasons stated below which will become apparent to those skilled in the art upon reading and understanding the present specification, there is a need in the art for a low cost, an efficient, and an effective system to measure moisture in the atmosphere.

### SUMMARY

The following summary is made by way of example and not by way of limitation. It is merely provided to aid the reader in understanding some of the aspects of the subject matter described. Embodiments provide an atmospheric moisture measuring system that detects moisture in the atmosphere using an aircraft air bleed system.

In one embodiment, an atmospheric moisture measuring system is provided that includes at least one moisture collector, at least one moisture sensor, at least one heater and at least one timer system. The at least one moisture collector is coupled to receive an airflow from a bleed system of a vehicle. Each moisture sensor is coupled to receive evaporated moisture from an associated moisture collector. Each heater is configured to heat an associated moisture collector to generate the evaporated moisture received by an associated moisture sensor. Each timer system is configured to selectively activate an associated heater.

In another embodiment a system to measure moisture in an atmosphere is provided. The system includes a first atmospheric moisture measuring system and a second moisture measuring system. The first atmospheric moisture measuring system includes a first moisture collector, a first moisture sensor, a first heater and a first timer system. The first moisture collector is coupled to receive an airflow from a bleed system of a vehicle. The first moisture sensor is coupled to receive first evaporated moisture from the first moisture collector. The first heater is configured to heat the first moisture collector to generate the evaporated moisture which is received by the first moisture sensor. The first timer system is configured to selectively activate the first heater at a first phase of a set frequency. The at least one second atmospheric moisture measuring system includes a second moisture collector, a second moisture sensor, a second heater and a second timer system. The second moisture collector is coupled to receive the airflow from the bleed system of the vehicle. The second moisture sensor is coupled to receive second evaporated moisture from the second moisture collector. The second heater is configured to heat the second moisture collector to generate the second evaporated moisture received by the second moisture sensor. The second timer system is configured to selectively activate the heater. The second timer system operates at a second phase of the set frequency.

In yet another embodiment, a method of measuring moisture in an atmosphere. The method includes collecting a sample of air from an airflow in a bleed system of a vehicle; turning on a heater for a select amount of time to generate evaporated moisture from the collected sample; and sensing a moisture level in the evaporated moisture with a moisture sensor.

### BRIEF DESCRIPTION OF THE DRAWINGS

The present invention can be more easily understood and further advantages and uses thereof will be more readily apparent, when considered in view of the detailed description and the following figures in which:
Figure 1 is a block diagram of a moisture measuring system according to an example aspect of the present invention;
Figure 2 is an illustration of a moisture measuring flow diagram according to an example aspect of the present invention; and
Figure 3 is an illustration of a moisture measurement implementation flow diagram according to an example aspect of the present invention.

In accordance with common practice, the various described features are not drawn to scale but are drawn to emphasize specific features relevant to the present invention. Reference characters denote like elements throughout Figures and text.

### DETAILED DESCRIPTION

In the following detailed description, reference is made to the accompanying drawings, which form a part hereof, and in which is shown by way of illustration specific embodiments in which the inventions may be practiced. These embodiments are described in sufficient detail to enable those skilled in the art to practice the invention, and it is to be understood that other embodiments may be utilized and that changes may be made without departing from the spirit and scope of the present invention. The following detailed description is, therefore, not to be taken in a limiting sense, and the scope of the present invention is defined only by the claims and equivalents thereof.

Embodiments of the present invention provide an atmospheric moisture measuring system that detects moisture in the atmosphere using a bleed system such as an aircraft air bleed system. The moisture measuring system in an example includes a collector, a heater, a detector and a timer system. The collector is used to gather moisture over a time period set by the timer system. Expiration of the time period activates the heater, or a similar device, which causes the collector to discharge the water molecules that have accumulated over the period set by the timer system. The discharge (evaporated moisture) is passed to a moisture sensor that generates an output signal of the detected moisture. In one example, a collection frequency and release cycle (heater activation) that provides a typical concentration factor of approximately 100X is used. This configuration provides a concentration factor that enables estimated detection requirements that align with the capabilities of multiple low-cost moisture sensors.

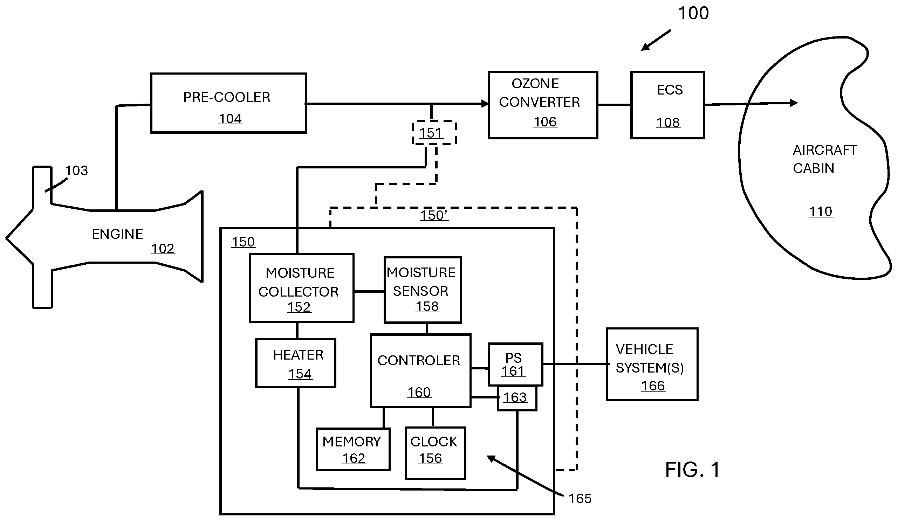

Referring to Figure 1, a block diagram of a simplified aircraft air bleed system 100 is illustrated. An air bleed system (vehicle air bleed system) uses pressurized air that is bled off of an airplane's turbine engine to pneumatic components, to for example, regulate cabin pressure, regulate air conditioning, prevent formation of ice on the wings of the aircraft, etc. The air bleed system 100 of Figure 1 includes a turbine engine 102 with a fan 103. Pressurized air is bled off to a pre-cooler 104. Pre-cooler 104 cools down the air from engine 102. The cooled air is then passed to an ozone converter 106. The Ozone converter 106 is placed in the ducting of the aircraft air bleed system to decompose any ozone in the air into oxygen. From the ozone converter 106, the air is passed to an environmental control system (ECS) 108. ECS 108 supplies the aircraft cabin 110 with air for cabin pressurization, thermal control and conditioned air, etc.

Further illustrated in Figure 1, is an atmospheric moisture measuring system 150 of an example embodiment. The atmospheric moisture measuring system 150 in this example includes a moisture collector 152 (H₂O collector), a moisture sensor 158 (H₂O sensor), a heater 154, a clock 156, a controller 160 and a memory 162. The moisture collector 152 is coupled to aircraft bleed system 100. In this example, the moisture collector 152 is coupled between the pre-cooler 104 and the ozone converter 106 to receive an airflow from the air bleed system 100. It may be desired to couple the moisture collector 152 before the ozone converter 106 because ozone converters are generally hygroscopic in that they tend to absorb moisture from the air. Hence, coupling the moisture collector 152 after the ozone converter 106 may result in inaccurate moisture measurements from the air. Examples of types of moisture collector. that may be used, include but are not limited to, a metal-organic frame (MOF) based collector and a chiller collector known in the art.

Heater 154 selectively heats the moisture collector 152 to cause the collected moisture to evaporate. The size of heater 154 is dependent on how rapidly the moisture needs to be released and the thermal mass of the moisture collected in the moisture collector 152. In one example, a simple resistance heater type is used. The evaporated moisture is received by moisture sensor 158 where the moisture is measured. Example types of moisture sensors 158 that may be used are metal oxide (metal oxide with an excess of electrons in the valence band) sensors, microelectromechanical system (MEMS) sensors, and nondispersive infrared sensors. Other types of moisture sensors may also be used. Heater activation, in one example, is controlled by a timer system 165.

The clock 156, in one example, may be a commercial off-the shelf clock for use in avionic applications. The timer system 165 may include clock 156, controller 160, and memory 162 in an example. In the example of Figure 1, a received clock output signal from clock 156 and operating instructions stored in memory 162 are used by controller 160 to selectively activate the heater 154.

In general, the controller 160 may include any one or more of a processor, microprocessor, a digital signal processor (DSP), an application specific integrated circuit (ASIC), a field program gate array (FPGA), or equivalent discrete or integrated logic circuitry. In some example embodiments, controller 160 may include multiple components, such as any combination of one or more microprocessors, one or more controllers, one or more DSPs, one or more ASICs, one or more FPGAs, as well as other discrete or integrated logic circuitry. The functions attributed to controller 160 herein may be embodied as software, firmware, hardware or any combination thereof. Controller 160 may be part of a system controller or a component controller. Memory162 may include computer-readable operating instructions that, when executed by controller 160 provide functions of the atmospheric moisture measuring system 150. Such functions may include the functions of selectively activating heater 154 as described above and functions of reading and processing an output of the moisture sensor 158 as described below. The computer readable instructions may be encoded within memory 162. Memory 162 is an appropriate non-transitory storage medium or media including any volatile, nonvolatile, magnetic, optical, or electrical media, such as, but not limited to, a random access memory (RAM), read-only memory (ROM), nonvolatile RAM (NVRAM), electrically-erasable programmable ROM (EEPROM), flash memory, or any other storage medium.

In an example, controller 160 activates a switch 163 that is positioned in a connection between a power supply 161 and the heater 154 to selectively activate the heater 154. An output of the moisture sensor 158, in one example, is fed to the controller 160. In other examples, the output may be fed into an avionic system of an aircraft via controller 160. Further, in an example, the controller 160 is part of an avionic system of the aircraft. In yet another example, the power supply 161 is connected to or is part of an aircraft system 166 and the controller 160, that is coupled to switch 163, provides the switch function.

Controller 160 may be in communication with one or more aircraft systems 166 in this example. Aircraft system(s) 166 (vehicle systems) may include a vehicle display system to display moisture detected, a vehicle communication system to communicate the detected moisture to a remote location where detected moisture levels are gathered and disseminated to other vehicles, as well as vehicle systems used to control operations of the vehicle. In one example, the detected moisture is used to one of determine if a different flight path should be implemented (i.e. for example, a flight path/altitude modification) or if operating parameters of the aircraft should be changed to reduce and/or avoid contrail formation. Further in an example, memory 162 is used to log moisture data from the moisture sensor 158. Further in an example, the logged data may be used for a remote download.

As discussed above, controller 160 is configured to selectively activate heater 154 to evaporate the moisture collected that is then measured by the moisture sensor 158. In one example, the activation of heater 154 is based on a phase of a set frequency and a duration by the controller 160 when instructed to measure the moisture in the air. The instructions may come from one of the aircraft systems 166, an operator input into one of the aircraft systems, or from the operating instructions stored in memory 162. The period between activations is at least in part dependent on the desired sensing range of atmospheric coverage as the vehicle passes through the atmosphere.

One factor in determining the duration of an activation of the heater 154 is the sensitivity level of the moisture sensor 158. The higher the sensitivity, the shorter the length of activation is needed. However, the higher the sensitivity of moisture sensor 158, the higher the cost of the sensor. Further, spacing the activations of the heater 154 too far apart results in capturing air across a wide distance of the atmosphere, therein reducing the sensing range of atmospheric coverage as the vehicle passes through the atmosphere. Accordingly, there is a trade-off between using cost effective moisture sensors and the frequency and length of activations of the heater 154.

In one example, a vehicle may use more than one atmospheric moisture measuring system 150 to increase the number of moisture measurement readings. For example, Figure 1 further illustrates a second atmospheric moisture measuring system 150'that would include the components of the atmospheric moisture measuring system 150. This arrangement provides a built in redundancy system and avoids the costs associated with having to use a highly sensitive moisture sensor to accomplish a narrow sensing range. Further, the use of multiple moisture measuring systems may also increase overall precision of moisture measurements and decrease errors. Further, in an example, different types of moisture sensors are used in the different atmospheric moisture measuring systems. The use of dissimilar technology in the different atmospheric moisture measuring systems may help guarantee the required sensitivity in a full flight envelope.

The first atmospheric moisture measuring system 150 and the second atmospheric moisture measuring system 150' may have staggered frequencies and/or phases of heater activation. In one example, a measurement from the first atmospheric moisture measuring system 150 is taken at a first phase of a set frequency and a measurement from the second atmospheric moisture measuring system 150 is taken at a second phase of the set frequency. Further, there may be more than one second atmospheric moisture measuring systems used to achieve a desired system of measuring moisture in the atmosphere (i.e., there may be 3, 4 or more atmospheric moisture measuring systems being used in a vehicle). Further in an example, moisture measurements from two or more atmospheric moisture measuring systems may be taken at a given time. In this example, an averaging implemented by the controller 160 may be used to improve precision of an overall determined moisture measurement of the system from the moisture measurements of the two or more atmospheric moisture measuring systems taken at the given time.

Further, in one example with multiple atmospheric moisture measuring systems being used, a timing switch 151 (or valve) is provided that is in a communication path between the aircraft bleed system 100 and the different moisture measuring systems 150 and 150'. The timing switch 151 is used to open or isolate paths between respective moisture collectors 152 in the different moisture measuring systems 150 and 150' and the aircraft bleed system 100. This allows a finite collection time to be measured at each moisture collector 152. One of the controllers 160 may be configured to control the timing switch 151. Further, the controller 160 used for the timing of timing switch 151 may be part of an aircraft system 166. Further in an example, the timing switch 151 may be used to selectively initiate a start of an air flow from the aircraft bleed system 100 to a moisture collector 152 of a moisture measuring system 150 or 150' when it is determined that moisture in the atmosphere should be measured.

In examples, moisture sensors typically implemented with standard integration/collecting times are implemented using longer collection times and longer activation times (e.g., for example, by a factor of 100X to reach a desired value). An example of a moisture sensor 158 that may be used in a moisture measuring system is a moisture sensor that can detect within a range of 600 ppm to 1200 ppm. In this example, heater 154 may be activated for a minimum of one second (which provides a concentration factor of approximately 100X). This provides a system sensitivity of 6 to 12 ppm for the atmospheric moisture measuring system 150.

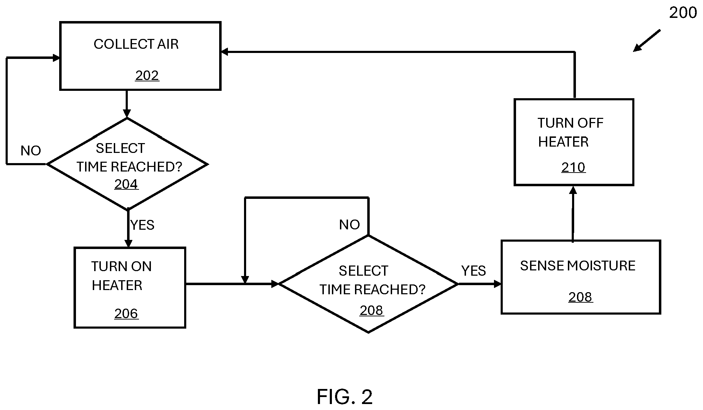

A method of operating the atmospheric moisture measuring system 150 is illustrated in a moisture measurement reading flow diagram 200 of Figure 2. The moisture measurement reading flow diagram 200 is provided in a series of sequential blocks. The sequence of blocks, however, may occur in a different order or even in parallel in other examples. Hence, the present invention is not limited to the sequential sequence of blocks as set out in Figure 2.

The moisture measurement reading flow diagram 200 in this example, starts a block 202 where moisture collector 152 collects a sample of moisture. It is determined at block 204 if a select amount of time has been reached in collecting the moisture. As discussed above, the select time depends on such factors as, but is not limited to, the sensitivity of moisture sensor 158 used in the atmospheric moisture measuring system 150, the desired breath of range (sensing range) of atmospheric coverage, and the speed of the vehicle.

If it is determined at block 204, the select amount of time has not passed, the process continues at block 202. If, however, it is determined at block 204 the select amount of time has passed, heater 154 is turned on at block 206. It is then determined at block 208 if a select amount of time has been reached for the heater to be activated. The duration selected may be determined by determining an amount of time needed to evaporate the moisture from the moisture collector 152. Once it is determined at block 208 the select amount of time has been reached, the moisture sensor 158, which is coupled to the moisture collector 152, measures the moisture level at block 208 and heater 154 is turned off at block 210. The process then continues at 202.

As discussed above, more than one atmospheric moisture measuring system 150 may be implemented staggering the frequencies of activation of the associated heaters. Further, as discussed above, at least one of the frequency and phase of activations of the heater 154 and the duration of activation of the heaters is determined by at least one of a sensitivity of the moisture sensor, a desired sensing range of atmospheric coverage as the vehicle travels, and speed of travel of the vehicle. The use of more than one atmospheric moisture measuring system 150 provides redundancy accuracy or measurement, validation of measurement, and a continuous data stream.

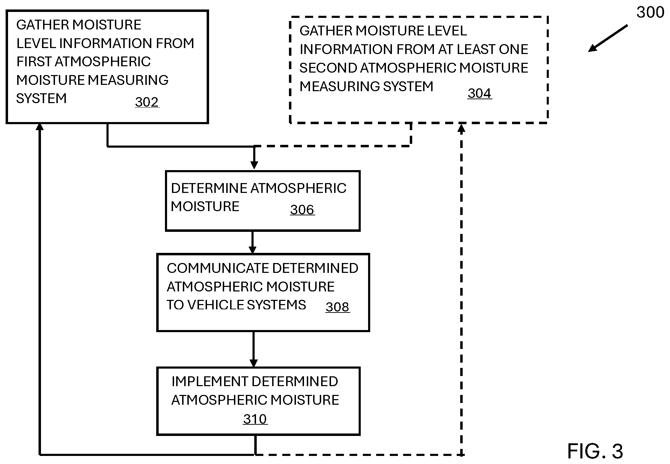

A method of implementing atmospheric moisture measurements is illustrated in a moisture measurement implementation flow diagram 300 of Figure 3. The moisture measurement implementation flow diagram 300 is provided in a series of sequential blocks. The sequence of blocks, however, may occur in a different order or even in parallel in other examples. Hence, the present invention is not limited to the sequential sequence of blocks as set out in Figure 3.

At block 302, first moisture level information is gathered from a first atmospheric moisture measuring system 150. If the system includes at least one other atmospheric moisture measuring system, second moisture level information is gathered from the at least one second moisture measuring system 150'. In one example, the first moisture level information is gathered at a different time than the second moisture level is gathered from at least one of the at least one second moisture measuring system 150'. One example, this is accomplished by selectively activating associated heaters 154 with one of frequency and phase activations as stated above. In another example, the first moisture level information is gathered at the same time as the second moisture level is gathered from at least one of the at least one second moisture measuring system 150'.

At block 306 the atmospheric moisture is determined. In an example with just one atmospheric moisture measuring system 150, this is simply the gathered moisture level information determined at block 302. In a system with more than one atmospheric moisture measuring system, in a different sampling time system discussed above, the determined atmospheric moisture is provided by then current moisture level information by either of the first atmospheric moisture measuring system 150 or the at one second atmospheric moisture measuring system 150'. In a system where both the first atmospheric moisture measuring system 150 and the at least one second atmospheric moisture measuring system 150' provides moisture level information at the same time, the moisture level information is averaged. In one example, a weighted average of the moisture level from the atmospheric moisture measuring systems is used to determine a moisture level. In a weighted average example, the moisture level is determined by weighing moisture levels based on one of sensitivity, accuracy, or even systematic errors associated with moisture measurement sampling rates, etc. In a system with a plurality of atmospheric moisture measuring systems, a first set of atmospheric moisture measuring systems may take measurements at a first phase while at least a second set of atmospheric moisture measuring systems may take measurements at a second phase. Hence, different sampling times with different combinations of atmospheric moisture measuring systems are contemplated.

At block 308, the determined atmospheric moisture is communicated to vehicle system(s) 166. The vehicle system(s) 166 then implements the determined atmospheric moisture at block 310 as discussed above. This dynamic process then continues at block 302 and 304 so the atmospheric moisture is continuously determined throughout a travel path (flight path).

### EXAMPLE EMBODIMENTS

Example 1 is an atmospheric moisture measuring system including at least one moisture collector, at least one moisture sensor, at least one heater and at least one timer system. The at least one moisture collector is coupled to receive an airflow from a bleed system of a vehicle. Each moisture sensor is coupled to receive evaporated moisture from an associated moisture collector of the at least one moisture collector. Each heater is configured to heat an associated moisture collector to generate the evaporated moisture received by an associated moisture sensor. Each timer system is configured to selectively activate an associated heater.

Example 2 includes the atmospheric moisture measuring system of Example 1, wherein the at least one moisture collector is coupled to selectively receive the airflow from between a pre-cooler and an ozone converter of the bleed system of the vehicle.

Example 3 includes the atmospheric measuring system of any of the Examples 1-3, wherein the at least one timer system further includes a clock, a memory, a power supply, a switch, and a controller. The clock is used to output a clock signal. The memory is used to at least store operating instructions and moisture measurements from the at least one moisture sensor. The switch is positioned between a connection between the power supply and the heater. The controller is configured to implement the operating instructions stored in the memory and receive the clock signal from the clock. The controller is further configured to control the switch based on the operating instructions in the memory to selectively activate the heater to heat the at least one moisture collector.

Example 4, includes the atmospheric moisture measuring system of any of the Examples 1-3, wherein a frequency of activations of the at least one heater and a duration of the activation of the at least one heater is dependent on at least one of a sensitivity of the at least one moisture sensor, a desired sensing range of atmospheric coverage as the vehicle travels, how rapidly the moisture needs to be released from the at least one moisture collector, a thermal mass of the moisture collected in the at least one moisture collector and a speed of travel of the vehicle.

Example 5 includes the atmospheric moisture measuring system of any of the Examples 1-4, wherein an output of the at least one moisture sensor is passed to at least one vehicle system of the vehicle.

Example 6 includes the atmospheric moisture measuring system of Example 5, wherein the at least one vehicle system is at least one of a first vehicle system to display a detected moisture level from the output of the moisture sensor, a second vehicle system to control operations of the vehicle, and a third vehicle system to communicate the detected moisture level to a remote location.

Example 7 includes the atmospheric moisture measuring system of any of the Examples 1-6, wherein the at least one sensor is a metal oxide sensor.

Example 8 includes the atmospheric moisture measuring system any of the Examples 1-7, wherein the at least one sensor includes one of a nondispersive infrared sensor and a capacitive type microelectromechanical system sensor.

Example 9 includes a system to measure moisture in an atmosphere. The system includes a first atmospheric moisture measuring system and a second moisture measuring system. The first atmospheric moisture measuring system includes a first moisture collector, a first moisture sensor, a first heater and a first timer system. The first moisture collector is coupled to receive an airflow from a bleed system of a vehicle. The first moisture sensor is coupled to receive first evaporated moisture from the first moisture collector. The first heater is configured to heat the first moisture collector to generate the first evaporated moisture which is received by the first moisture sensor. The first timer system is configured to selectively activate the first heater at a first phase of a set frequency. The at least one second atmospheric moisture measuring system includes a second moisture collector, a second moisture sensor, a second heater and a second timer system. The second moisture collector is coupled to receive the airflow from the bleed system of the vehicle. The second moisture sensor is coupled to receive second evaporated moisture from the second moisture collector. The second heater is configured to heat the second moisture collector to generate the second evaporated moisture received by the second moisture sensor. The second timer system is configured to selectively activate the second heater. The second timer system operates at a second phase of the set frequency.

Example 10 includes the system of Example 9, further including at least one timing switch to selectively couple one of the first moisture collector of the first atmospheric moisture measuring system and the second moisture collector of the at least one second atmospheric moisture measuring system to the bleed system of the vehicle.

Example 11 includes the system of any of the Examples 9-10, wherein the first atmospheric moisture measuring system is configured to generate a first moisture measurement at a given time and the at least one second atmospheric moisture measuring system is configured to generate at least a second moisture measurement at the given time.

Example 12 includes the system of Example 11, further including at least one controller configured to use a weighted average of the first moisture measurement and the at least one second atmospheric moisture measurement in determining a moisture measurement.

Example 13 includes the system of any Examples 9-12, wherein at least one of the first sensor moisture and the second sensor moisture is metal oxide sensor.

Example 14 includes the system of any Example 9-13, wherein at least one of the first moisture sensor and the second moisture sensor is one of a nondispersive infrared sensor, and a capacitive type microelectromechanical system sensor.

Example 15 includes a method of measuring moisture in an atmosphere. The method includes collecting a sample of air from an airflow in a bleed system of a vehicle; turning on a heater for a select amount of time to generate evaporated moisture from the collected sample; and sensing a moisture level in the evaporated moisture with a moisture sensor.

Example 16 includes the method of Example 15, further including positioning a moisture collector to receive the airflow in the bleed system to collect the sample of air.

Example 17 includes the method of any of the Examples 15-16, wherein positioning the moisture collector further includes positioning the moisture collector between a pre-cooler and ozone converter of the bleed system.

Example 18 includes the methos of any of the Examples 15-17, further including using a timer system to determine the select amount of time.

Example 19 includes the method of any of the Examples 15-18, wherein the select amount of time is dependent on at least one of a sensitivity of the moisture sensor a desired sensing range of atmospheric coverage as the vehicle travels, how rapidly the moisture needs to be released from a moisture collector, a thermal mass of the moisture collected in the moisture collector and a speed of travel of the vehicle.

Example 20 includes the method of any of the Examples 15-19, further including activating the heater based on a select frequency.

Although specific embodiments have been illustrated and described herein, it will be appreciated by those of ordinary skill in the art that any arrangement, which is calculated to achieve the same purpose, may be substituted for the specific embodiment shown. This application is intended to cover any adaptations or variations of the present invention. Therefore, it is manifestly intended that this invention be limited only by the claims and the equivalents thereof.

## Claims

1. An atmospheric moisture measuring system (150) comprising:
at least one moisture collector (152) coupled to receive an airflow from a bleed system (100) of a vehicle;
at least one moisture sensor (158), each moisture sensor (158) coupled to receive evaporated moisture from an associated moisture collector (152) of the at least one moisture collector (152);
at least one heater (154), each heater (154) configured to heat an associated moisture collector (152) to generate the evaporated moisture received by the associated moisture sensor (152); and
at least one timer system (165), each timer system (165) configured to selectively activate an associated heater.

2. The atmospheric moisture measuring system (150) of claim 1, wherein the at least one moisture collector 152 is coupled to selectively receive the airflow from between a pre-cooler (104) and an ozone converter (106) of the bleed system (100) of the vehicle.

3. The atmospheric moisture measuring system (150) of claim 1, wherein the at least one timer system (165) further comprises:
a clock (156) to output a clock signal;
a memory (162) to at least store operating instructions and moisture measurements from the at least one moisture sensor (158);
a power supply (161);
a switch (163) positioned between a connection between the power supply (161) and the heater (154); and
a controller (160) configured to implement the operating instructions stored in the memory (162) and receive the clock signal from the clock (156), the controller (160) further configured to control the switch (163) based on the operating instructions in the memory (162) to selectively activate the heater (154) to heat the at least one moisture collector (152).

4. The atmospheric moisture measuring system (150) of claim 1, wherein a frequency of activations of the at least one heater (154) and a duration of the activation of the at least one heater (154) is dependent on at least one of a sensitivity of the at least one moisture sensor (158), a desired sensing range of atmospheric coverage as the vehicle travels, how rapidly the moisture needs to be released from the at least one moisture collector (152), a thermal mass of the moisture collected in the at least one moisture collector (152) and a speed of travel of the vehicle.

5. The atmospheric moisture measuring system (150) of claim 1, wherein an output of the at least one moisture sensor (158) is passed to at least one vehicle system (166) of the vehicle.

6. The atmospheric moisture measuring system (150) of claim 5, wherein the at least one vehicle system (166) is at least one of a first vehicle system to display a detected moisture level from the output of the moisture sensor (158), a second vehicle system to control operations of the vehicle, and a third vehicle system to communicate the detected moisture level to a remote location.

7. The atmospheric moisture measuring system (150) of claim 1, wherein the at least one moisture sensor (158) is one of a metal oxide sensor, a nondispersive infrared sensor, and a capacitive type microelectromechanical system sensor.

8. A method of measuring moisture in an atmosphere, the method comprising:
collecting a sample of air from an airflow in a bleed system (100) of a vehicle;
turning on a heater (154) for a select amount of time to generate evaporated moisture from the collected sample; and
sensing a moisture level in the evaporated moisture with a moisture sensor (158).

9. The method of claim 8, further comprising:
positioning a moisture collector (152) to receive the airflow in the bleed system (100) to collect the sample of air.

10. The method of claim 8, wherein positioning the moisture collector (152) further comprising:
positioning the moisture collector (152) between a pre-cooler (104) and ozone converter (106) of the bleed system (100).
